# EUROPEAN PATENT APPLICATION

(11) **EP 0 897 712 A1**
(43) Date of publication of application: **24.02.1999**
(21) Application number: 98115823.1
(22) Date of filing: 21.08.1998
(51) Int. Cl.: A61K 7/06

(54) **Cosmetic composition**

(30) Priority: 21.08.1997 JP 239143/97
(71) Applicant: SHISEIDO COMPANY LIMITED, Chuo-ku, Tokyo 104-8010 (JP)
(72) Inventor: Ota, Masahiro, c/o Shiseido Research Center (1), Yokohama-shi, Kanagawa-ken 223-8553 (JP); Okazaki, Tomomi, c/o Shiseido Research Center (1), Yokohama-shi, Kanagawa-ken 223-8553 (JP); Kobayashi, Koji, c/o Shiseido Research Center (1), Yokohama-shi, Kanagawa-ken 223-8553 (JP); Tajima, Masahiro, c/o Shiseido Research Center (1), Yokohama-shi, Kanagawa-ken 223-8553 (JP); Iwabuchi, Tokuro, c/o Shiseido Research Center (1), Yokohama-shi, Kanagawa-ken 223-8553 (JP); Ishino, Akihiro, c/o Shiseido Research Center (1), Yokohama-shi, Kanagawa-ken 223-8553 (JP); Tsuji, Yoshiharu, c/o Shiseido Research Center (1), Yokohama-shi, Kanagawa-ken 223-8553 (JP); Magara, Tsunao, c/o Shiseido Research Center (1), Yokohama-shi, Kanagawa-ken 223-8553 (JP)
(74) Representative: Kraus, Walter, Dr.

(57) **Abstract**

This specification relates to a cosmetic composition containing art extract of Croton birmanicus as an effective ingredient, particularly, a hair-growing composition. The composition not only has excellent hair revitalizing actions such as an alopecia preventing effect and a hair generation accelerating effect, and a dandruff or itch inhibiting action, but also has a specific hair growth accelerating action through activation of hair dermal papilla cells or hair follicular epitheliocytes.

## Description

### BACKGROUND OF THE INVENTION

This invention relates to use of an extract from a plant, particularly a plant used as a specific crude drug for a composition for human head.

In contemporary society called society of advanced age or society of stress, such opportunities that hair at the head is exposed to the crisis of alopecia due to various causes are more and more increasing.

In accordance therewith, various attempts are being made for providing further excellent "hair-revitalizing agents".

As chief effects given to hair by hair-revitalizing agents, there can be mentioned effects such as a hair generation-inducing effect (hair generation-accelerating effect, anagen-inducing effect), an effect to thicken hair, a hair anagen-lengthening effect, a 5a-reductase-inhibiting effect, a blood circulation-accelerating effect, a sterilizing effect, a dandruff-preventing effect, a moisturizing effect and an antioxidant effect. In methods for screening substances having such effects, prevention of alopecia, hair generation accelerating effect, or dandruff or itch inhibiting action has, generally, been used as an index.

However, although various attempts are being made, as mentioned above, conventional hair-revitalizing agents have not always been satisfactory in hair revitalizing actions such as prevention of alopecia and hair generating effect. It is understood that the reason is that, probably, alopecia occurs by various causes and the mechanism of hair generation is very complicated. Therefore, it is a more promising means to create a hair-revitalizing agents fixing eyes on the more specific structure itself of hair, and the provision of effective ingredients thus developed will be desired.

Thus, the object of the invention lies in providing a composition for the head not only having comprehensively an alopecia preventing effect, a hair generation accelerating effect and a dandruff or itch inhibiting action, but also being able to act directly on more specific structure (or construction) of hair. particularly hair dermal papilla cells or hair follicular epitheliocytes.

### SUMMARY OF THE INVENTION

The present inventors have, first, made researches for establishing a screening system or evaluation test system for effective ingredients capable of accomplishing the above object. As a result, they found that test substances bringing about good results when used in a cell proliferation test using human being-derived cultured hair dermal papilla cells or an activation test for human being-derived cultured hair follicular epitheliocytes, details of which are described hereinafter, not only have an effect to activate hair papilla (or hair dermal papilla cells) or lengthen the hair anagen in the hair cycle, but also can have a significantly excellent hair generation accelerating effect, hair growing effect, etc.

They further subjected extracts of various plants to both tests, and found that extracts from plants being classified as the genus Croton of the family Euphorbiaceae have excellent effects, and show an excellent hair revitalizing action such as an alopecia preventing effect or a hair generation accelerating effect and a dandruff or itch inhibiting action.

Therefore, according to the invention is provided a composition for the head containing an extract from a plant belonging to the genus Croton of the family Euphorbiaceae as an effective ingredient. As more specific embodiment is provided a hair-revitalizing composition comprising an extract from a plant belonging to the genus Croton of the family Euphorbiaceae in an amount effective for revitalization of human hair, and cosmetically or dermatologically acceptable other additives.

Further, as another embodiment of the invention is also provided the above-mentioned extract for use as an effective ingredient for hair revitalizing compositions, or a hair revitalization method using the extract.

Further, as still another embodiment of the invention is also provided a composition for activating hair dermal papilla cells and/or lengthening the hair anagen in the hair cycle which contains the extract as an effective ingredient.

### BRIEF DESCRIPTION OF THE DRAWINGS

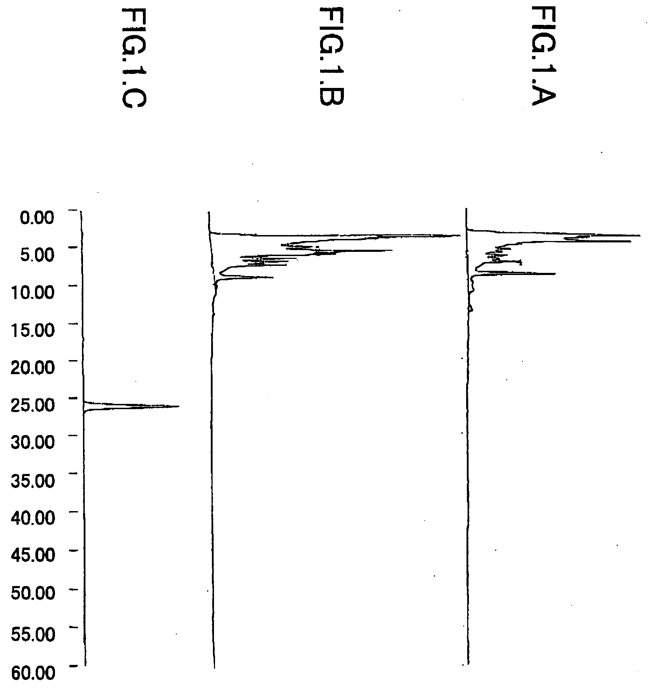

Fig. 1 A-C show comparatively the HPLC elution patterns of 4α-TPA on the specific fractions which were obtained from the extract derived from Croton birmanicus Muell. Arg., according to the invention and have heightened concentrations of the effective ingredients. Fig. 1A and Fig. 1B are Fraction A and Fraction B obtained "other preparation examples", respectively, and Fig, 1C is 4α-TPA for comparison.

### DESCRIPTION OF THE PREFERRED EMBODIMENT

The composition for the head in the invention includes compositions of any forms which can be used onto human heads, more specifically hairs and scalps Such compositions can widely be used for hair revitalization, and in the present specification, "hair revitalization" is used in a conception to include acceleration of hair generation, prevention of alopecia, an action to inhibit dandruff or itch, etc., and further, an action to activate papillary cells or hair follicular epitheliocytes.

Although not limited by the theory, an extract from a plant belonging to the genus Croton of the family Euphorbiaceae as an effective ingredient used in the invention has a hair papilla activation action because it significantly proliferates human being-derived cultured hair dermal papilla cells, and, moreover, also has an action to lengthen the anagen of hair in the hair cycle because it maintains or accelerates proliferation of human being-derived cultured hair follicular epitheliocytes, as stated later.

Therefore, the extract has hair generation accelerating and alopecia preventing effects by acting directly on the structure (or constituents) itself of hair, and further also has a dandruff or itch inhibiting action because it shows its efficacy without having a bad influence on proliferation of the above cells.

Particualrly, these effects and actions are surprising taking it into account that Croton oil obtained from germinal milk of seeds of Croton tiglium L., a plant belonging to the genus Croton of the family Euphorbiaceae (Tiglii Semen or croton seed) by pressing only has hair generating and hair growing actions significantly lower than the extract of the invention, and, on the other hand, has a skin stimulating action.

It is well-known that Croton oil contains 12-O-tetradecanoyl-phorbol 13-acetate (hereinafter, referred to as TPA) known as a tumor promoter which served as a basis of the establishment of the two-stage theory of a tumor promoter. This TPA is known to have a strong hair generating effect in C3H mice ("normal and Abnormal Epidermal Differentiation", edited by M. Seiji and I.A. Bernstein, pages 159-170, published in 1982 by Todai-Shuppan), but induces strong inflammation by topical application onto the ears of mice (P.L. Stanley et al., Skin Pharmacol. 1991; 4: 262-271) and brings about serious changes of epidermic cells (e.g., acanthosis, hyperkeratosis, etc.) by application onto the skin of Balb/c mice.

When such a background is taken into account, it is unexpected that the extract according to the invention shows significantly excellent hair generation accelerating effect and hair growing effect, and, moreover, hardly shows skin stimulating properties, and thus the usefulness of the screening or evaluation test method is proved which was established by the present inventors and uses cultured hair dermal papilla cells and cultured hair follicular epitheliocytes,

The extract used in the invention has its characteristic in the point that it is not a pressed oil from the germinal milk of seeds of the genus Croton of the family Euphorbiaceae, but obtained by extraction treatment.

Plants from which the extract of the invention can be obtained may be ones belonging to any species so long as they are plants being classified as the genus Croton and plants from which an extract meeting the object of the invention can be obtained. However, as preferred ones, there can be mentioned Croton birmanicus Muell. Arg. The plant is distributed chiefly in South-east Asia, and called HADSAKHYYN in Thailand.

The extract used in the invention can be obtained by immersing in an extracting solvent or refluxing with heating together with an extracting solvent leaves, stems including subterranean stems, roots, fruits, bark, etc. of the plant or the whole plant, subjecting the resutant mixture to filtration, and concentrating the filtrate. The extracting solvent can be any one so long as it is a solvent usually used for extraction, and, particularly, there can be mentioned one or a mixture of two or more selected from water-miscible organic solvents, for example, lower alcohols such as methanol, ethanol, isopropanol and n-butanol, polyhydric alcohols such as propylene glycol and 1,3-butylene glycol, acetone, ethyl acetate ester, etc. When a lower alcohol is used, the resultant extract can be used as such, but it is also possible to distil off the extracting solvent and incorporate the residue, if necessary after being dried, to give a composition for the head of the invention.

The extract according to the invention can be a treated product at each stage, in accordance with the object, obtained by further subjecting an extract obtained using the above extracting solvent to liquid-liquid distribution with a system comprising a hydrophobic solvent (e.g., n-hexane) and water, extracting the aqueous layer, for example, with ethyl acetate or the like, and then subjecting the extract or the above aqueous layer without the extraction to a suitable column chromatography treatment for separation or the like.

The compounding amount of the extract according to the invention into the composition for the head is not specified because it can vary depending on the forms or use methods of the composition. However, when such an extract as obtained according to the method described in the later-described examples is used, the compounding amount based on the whole weight of the composition is generally 0.0005 to 20.0 % by weight, preferably 0.001 to 10.0 % by weight in terms of dried matter. When it is more than 20.0 % by weight, formulation is difficult, which is undesirable. Further, even if it is compounded in an amount of more than 10.0 % by weight, so much increase of effect cannot be obtained.

There can be compounded, appropriately according to necessity, into the composition for the head of the invention, besides the above indispensable ingredient, cosmetically or dermatologically acceptable other additives, or components usually used in compositions for the head of cosmetics, quasi-drugs, drugs, etc., for example, oily components, humectants, thickening agents, sequestering agents, other active components, antioxdants, ultraviolet absorbers, surfactants, alcohols, powder components, coloring agents, aqueous components, water, various nutrients for the skin, etc.

Specifically, there can be mentioned, e.g., higher fatty acids, solid paraffins, liquid paraffin, silicone oils, squalane, etc. as oily matter: e.g., hyaluronic acid, propylene glycol, maltitol, atherocollagen, sodium lactate, etc. as humectants; e.g., quince viscous matter, carboxyvinyl polymers, xanthane gum, etc. as thickening agents; e.g., disodium edetate, trisodium edetate, sodium citrate, sodium polyphosphate, sodium metaphosphate and gluconic acid as sequestering agents; e.g., drugs such as caffeine, tannin, verapamil, tranexamic acid and its derivatives, glycyrrhiza extract, glabridin, extract of fruit of Trapa iaponica Flerov. with hot water, various crude drugs, tocopherol acetate, and glycylrrhetinic acid and its derivatives or its salts, other whitening agents such as vitamin C, magnesium phosphoascorbate, ascorbic acid glucoside, arbutin and kojic acid, saccharides such as glucose, fructose, mannose, sucrose and trehalose, etc., as other active components.

Further, as auxiliary components of hair revitalizing agents, there can simultaneously be compounded, in preparation of these formulations, in addition to the above oily components, humectants and thickening agents, for example oily matter such as glyceryl monooleate, vasodilators such as nicotinic acid amide, benzyl nicotinate, vitamin E acetate, Swertia Herb extracts, carpronium chloride and acetylcholine derivatives, amino acids such as serine and methionine, vitamins such as vitamin B6, vitamin E and its derivatives and biotin, skin function accelerators such as pantothenic acid and its derivatives, glycylrrhetinic acid and its derivatives, nicotinic acid, nicotinic esters such as methyl nicotinate and tocopherol nicotinate, and cepharanthine, female hormones such as estradiol, etc.

Further, there can suitably be compounded, in such a range that the effects of the invention are not spoiled, additives usually used in hair revitalizing agents, for example, antibacterial agents such as hinokitiol, hexachlorophene, benzalkonium chloride, cetylpyridinium chloride, undecylenic acid, trichlorocarbanilide and bithionol, refreshing agents such as menthol, drugs such as salicylic acid, zinc and its derivatives, and lactic acid and its alkyl esters, organic acids such as citric acid, amino acids such as arginine, etc.

The form of the compositin of the invention can be forms such as, for example, tonics, hair creams, mousses, shampoos and rinses.

### Examples

The invention is further specifically described below according to examples, but the invention is not limited thereby. The compounding amounts are % by weight, unless otherwise noted.

First, test methods and test results relating to ① hair generating effect, ② hair growing effect, ③ cellular proliferation using culture hair dermal papilla cells and ④ activation of human cultured hair follicul ar epitheliocytes are described in this order.

### ① Hair generation test

### 1. Preparation of extract (Preparation example 1)

Croton birmanicus (dried matter) (500 g) was immersed in 5.0 liters of methanol at room temperature for 5 days. The solvent was distilled off from the extract, and the resultant matter was dried to give 15.2 g of a dried methanol extract of Croton birmanicus.

### 2. Preparation of samples

The thus obtained dried methanol extract was dissolved in 75% ethanol solution so that the concentrations could be 0.5%, 1% and 2%, to give Samples 1 to 3.

### 3. Hair generation test method and its results

The following hair generation test was carried out on Samples 1 to 3 in a liquid state of the invention, using as a control sample 75% ethanol solution of 0.1% Croton oil.

The test was carried out by the method of Ogawa et al., "Normal and Abnormal Epidermal Differentiation". edited by M. Seiji and I.A. Bernstein, pages 159-170, published in 1982 by Todai Shuppan) using as an laboratory animal C3H/HeNCrJ mice in the telogen of the hair cycle.

Namely, the mice were divided into 4 groups for Test samples 1 to 3 and Control sample, each group consisting of 10 animals. The back of each mouse was shaved by hair clippers and a shaver, and each sample was applied in o.1 ml portions once a day. 18 days and 24 days thereafter, the area where hair was regenerated was measured. The results were expressed as an average value of the regenerated areas. The results are shown in Table 1.

**Table 1**

| | Hair regeneration area (%) | |
|---|---|---|
| Sample | 18 days later | 24 days later |
| Sample 1 (Croton birmanicus extract 0. 5 %) | 76 | 96 |
| Sample 1 (Croton birmanicus extract 1 %) | 87 | 100 |
| Sample 1 (Croton birmanicus extract 2 %) | 92 | 100 |
| Control sample | 37 | 66 |

As apparent from Table 1, the Croton birmanicus extract of the invention showed excellent hair generating effect in the murine hair generation test. 2. Other preparation examples

A methanol extract obtained in the same manner as in Preparation example 1 (299.2 g) (dry weight) was subjected to liquid-liquid distribution between n-hexane (6 L) and water (3 L) to give 51.83 g of dried matter from the n-hexane layer and give the aqueous layer. The aqueous layer was subjected to liquid-liquid distribution between it and ethyl acetate (10 L), and 52.7 g of dried matter was obtained from the ethyl acetate layer (hereinafter, referred to as ethyl acetate fraction).

The ethyl acetate fraction was fractionated through silica gel column (packing material; Wakogel C-200: 1 kg, eluent ; chloroform:methanol = 50:1 → 30 : 1→20 : 1→9 : 1→2 : 1), the resultant fractions on which C3H mouse hair generating effect was detected were further fractionated by ODS gel column chromatography (ODS gel: 300 g, eluent ; 70% methanol→90% methanol→ 100% methanol). and 1. 10 g of dried matter was obtained from the middle-stage eluate fractions on which C3H mouse hair generating effect was detected. This was fractionated through silica gel column (packing material: Wakogel C-200: 170 g, eluent : chloroform:methanol = 20:1→10:1→5:1→2:1→1:1→0:1) From the initial-stage eluate fraction to about 2/5 eluate fraction was obtained 520 mg of dried matter (hereinafter, referred to as Fraction A), and from the 3/5 eluate fraction was obtained 530 mg of dried matter (hereinafter, referred to as Fraction B.

HPLC was carried out under the following conditions on Fraction A and Fraction B obtained above and 4α-TPA (comparison). The resultant elution patterns of the respective components are shown in comparison in Fig. 1. A-C.

### HPLC conditions

| Mobile phase |
|---|
| 0-50 minutes: 80% acetonitrile |
| 50-60 minutes: 80→100% acetonitrile |
| 60-80 minutes: 100% acetonitrile |

| Column |
|---|
| Capcell Pak C18 UG120 Å |

| Detection |
|---|
| UV 238 nm |

These samples were subjected to the above hair generation test, and further, stimulation was observed on the skin of the back parts of C3H/HeNCrJ mice which were shaved and coated with the samples. This stimulation means desquamation and acanthosis, and when these are remarkably observed, the expression of ++ is made. when observed to some degree, +, and when not observed at all, -.

As to hair generating effect, when the hair regeneration area is 80% or more and 100% or less, the expression of +++ is made, when 60% or more and less than 80%, ++, and when 40% or more and less than 60%, +.

The results of the test are shown in the following Table 2 (the sample concentration was adjusted with 75% ethanol).

**Table 2**

| Sample | | Hair generating effect | Stimulation |
|---|---|---|---|
| Extract of Preparation example 1 | 0.5 % | ++ | - |
| Ethyl acetate fraction | 0.5 % | +++ | - |
| Fraction A | 0.1 % | +++ | - |
| Fraction B | 0.1 % | +++ | - |
| TPA | 6 ppm | +++ | ++ |
| TPA | 0. 6 ppm | +++ | ++ |
| Croton oil | 0.1 % | + | + |

It is understood, as stated in the above literature, that TPA shows remarkable skin stimulation properties.

### ② Hair growth test (trichogram test)

### 1. Preparation of extract

An extracts was prepared according to the method of Preparation example 1 in the above hair generation test example.

### 2. Preparation of samples

The dried methanol extract obtained in the above method (0.5, 1 or 2%) was mixed under stirring with 70% ethanol solution (90%), sodium oleate (0. 01%), dodecylbenzenesulfonic acid (0.49%), hardened castor oil ethylene oxide (40 mol) adduct (0.5%) and deionized water (8%) to give a solution. Further, deionized water (residual part) was mixed therewith to give Samples 4, 5 and 6 in a liquid state.

### 3. Hair growth test method and its results

In order to examine the hair revitalizing actions such as prevention of alopecia and hair generating effect of the composition for the head of the invention, a trichogram test was carried out as follows on human beings.

The hair roots of hairs pulled out before and after the use of each sample were observed under a microscope, the number of hair roots at the telogen was counted based on form of the hair roots, and hair revitalizing action was compared by the increase or decrease of the rate. Hair roots at the telogen are hair roots of hairs whose growth stopped, and it is recognized that persons complaining of alopecia have a higher rate of hair roots at the telogen than normal persons.

Each of the test samples and the control sample was applied onto the scalps of 10 male subjects per sample twice a day, in an amount of 2 ml per one application, for continuous 6 months. 100 Hairs per each subject were pulled out immediately before the application and immediately after the completion of the 6 months application, respectively, and the respective hair roots were examined, and thereby an actual use test was carried out. The results are shown in Table 3.

**Table 3**

| Sample | Rate of hair roots at the telogen | | | Evaluation of hair revitalizing effect |
|---|---|---|---|---|
| | 20% or more decrease | ±20% | 20% or more increase | |
| Sample 4 (0.5%) | 59 | 33 | 10 | effective |
| Sample 5 ( 1%) | 72 | 32 | 1 | remarkably effective |
| Sample 6 ( 2%) | 80 | 20 | 0 | remarkably effective |
| Control sample | 9 | 31 | 59 | noneffective |

As apparent from Table 3, the Croton birmanicus extract of the invention showed a significant hair growing effect in the human trichogram test.

### ③ Cell proliferation test using cultured hair dermal papilla cells

### 1. Preparation of extract

The preparatin of the above extract was carried out by the method of Preparation method 1 in the above hair generation test. Further, the dried methanol extract was made into a 0.2% by weight solution with DMSO, and this was diluted with a serum-free medium (MEM) to give solutions containing the Croton birmanicus extract in concentrations of 1.0⁻⁹ x 10 to 1.0 x 10⁻⁶ % (Preparation examples 2 to 5).

### 2. Collection of hair dermal papilla cells

The fatty tissue was separated from the skin (5 mm x 1.5 cm) at the occipital region of head of 32-year-old male extirpated by art orthopedic operation, hair follicles were extirpated therefrom, and hair dermal papilla cells were isolated from the hair bulb part. The isolated hair dermal papilla cells were cultured for 2 weeks [37°C, 5% CO₂] in MEM containing 20% FBS. At the time when the outgrowth of cells from the hair dermal papilla cells was confirmed, the medium was replaced with MEM containing 10% FBS (MEM + 10% FBS), and culturing was carried out under the same conditions. Thereafter, the medium was replaced with MEM + 10% FBS at a rate of twice a week to maintain the cells.

4 weeks after the start of culturing, subculturing was carried out, and thereafter, at the time when the cells sufficiently proliferated, subculturing was carried out again, and this passage was repeated.

### 3. Test method

A cell suspension having a cell density of 10,000 cells/ml was prepared using hair dermal papilla cells of passage number 3 and MEM+10% FBS medium. 200 µl portions of this cell suspension were put in a 96-well microplate (namely, 2,000 cells/well), and incubation was carried out at 37°C and 5% CO₂ for 3 days to let the cells adhere. 72 Hours after the start of culturing, the culture broth for control was replaced with serum-free MEM, and the culture broths for test sample systems were replaced with serum-free media (MEM) containing the Croton birmanicus extract (Preparation examples 2 to 5) (Samples 7 to 10).

The control and test sample systems were cultured for further 4 days.

After the completion of culturing, 20 µl of alamar blue (made by BIOSOURSE) was added to each system, culturing was carried out for further 8 hours, and absorbance was measured at 570 nm and 595 nm by Micro plate reader (made by BIO RAD). According to the attached operation manual, the reduction rate of alamar blue was calculated based on the measurement results of absorbance. Since this reduction rate correlates with cell number, the cell proliferation rates in the control and test sample systems were compared.

### 4. Results

The cell proliferation acceleration indexes in the measured Croton birmanicus extracts (Samples 7 to 10) are shown in the following Table 4.

**Table 4**

| Sample | Proliferation acceleration rate (%) | Effect |
|---|---|---|
| Sample 7 (1.0 x 10⁻⁹ %) | 4.6±3.7* | effective |
| Sample 8 (1.0 x 10⁻⁸ %) | 18.7±6.0** | effective |
| Sample 9 (1. 0 x 10⁻⁷ %) | 13.5±5. 4** | effective |
| Sample 10 (1.0 x 10⁻⁶ %) | 6.3±3.2 | noneffective |
| Control sample | 2.1±3.1 | noneffective |

| | | |
|---|---|---|
| *; significant difference p<0. 05 | | |
| **; significant difference p<0.01 | | |

As apparent from Table 4, the Croton birmanicus extract of the invention showed a significant hair papilla cell proliferation accelerating effect in the cultured hair papilla cell activation test.

### ④ human cultured follicular epitheliocyte activation test

### 1. Collection of human cultured follicular epitheliocyte

Hair follicles at the anagen in the hair cycle were mechanically collected under a stereoscopic microscope from a human male scalp obtained as a by-product of a surgical operation. These hair follicles at the anagen were treated at 37°C for 30 minutes with Dulbecco's modification of MEM (DMEM) containing 1,000 U/ml dispase and 0. 2% collagenase, the dermal sheath and the dermal papilla and the epithelial tissue at the hair bulb part were removed using the tip of an injection needle, and the resultant matter was treated at 37°C for 5 minutes with phosphate buffer [PBS (-); (-) means not containing calcium ions and magnesium ions] containing 0.05% trypsin and 0.02% EPTA.

Then, the hair follicles were allowed to stand in a culture plate coated with collagen (Type I), and explant culturing was carried out. As the medium was used a serum-free medium [Keratinocyte Growth Medium (KGM)] (Keratinocyte Serum Free Medium can also be used).

4 to 5 Days after this culturing, at the time when adhesion of the hair follicles onto the culture plate and proliferation of the cells were confirmed, the medium was replaced, and thereafter, medium replacement was carried out every two days.

The thus proliferated cells were treated at 37°C for 5 minutes with 0.05% by weight trypsin - 0.02% EDTA, the reaction was stopped with an equal volume of 0.1% trypsin inhibitor, and the cells were collected by centrifugation (800 x g, 5 minutes).

Then, the cells were floated in the same serum-free medium as above, the suspension was spread with a density of 5.000 cells/cm² on a culture plate coated with collagen (Type I), medium replacement was carried out every two days until the cells became subconfluent, the cells were treated again at 37°C for 5 minutes with 0.05% by weight trypsin - 0.02% EDTA, the reaction was stopped with an equal volume of 0, 1% of the trypsin inhibitor, centrifugation (800 x g, 5 minutes) was carried out, a cell-freezing liquid (Cell Banker: made by DIA-IATRON) was added to the resultant human hair follicular epitheliocytes to adjust the cell concentration to 1.0 x 10⁵ cells/ml), 1.0 x 10⁶ cells portions of the cells were put in freezing tubes, respectively and they were preserved under freezing, These cell numbers were calculated by a cytometer.

The fibroblast contamination rate (FB contamination rate) of the hair follicular epitheliocytes obtained by the above step was measured (3,000 magnifications, 5 visual fields), and the cells having a FB contamination rate of 3% or more were excluded from the subjects of the assay.

The remaining hair follicular epitheliocytes were spread in a culture flask, they were treated with 0.05% trypsin and 0.02% EDTA, the reaction was stopped with 0.1% of the trypsin inhibitor, the system was subjected to centrifugation at 1.500 rpm for 5 minutes. the supernatant was removed, and 20 ml of KGM medium was added to the residue to give a cell suspension.

The cell suspension was spread on a 96-well plate (plate coated with I-type collagen: made by FALCON) at a rate of 0.2 ml/well (1.0 x 10⁴ cells/well), and the system was left alone at room temperature for about 20 minutes until the cells sank on the bottom of the wells.

Thereafter, culturing was carried out at 37°C and 5% CO₂ for one day to give desired human cultured hair follicular epitheliocytes.

5 ml of PBS (-) containing 0.25% trypsin was added to the hair follicles obtained by the above operations, and the cell suspension was incubated at 37°C for 5 minutes.

After completion of the incubation, 5 ml of fetal bovine serum (FBS) and 5 ml of Ham's F12 medium were added, the cell suspension was filtered by Cell Strainer (100 µm, made by NALGENE) and put in a 50-ml centrifugation tube, and the cell suspension was centrifuged (4°C, 1,500 rpm, 5 minutes). The supernatant was removed from the system, and desired hair follicular epitheliocytes were obtained as the residue.

### 2. Preculturing of the hair follicular epitheliocytes

In order to remove as many fibroblasts as possible contaminating the system, preculturing of the hair follicular epitheliocytes obtained by the above step was carried out. The procedure is described below.

The freezed cells obtained in the above step were thawed out in a constant temperature vessel of 37°C. Then, 10 ml of FAD medium [a medium obtained by mixing Ham's F12 medium (described later) and MEN medium in a volume ratio of 3:1 and adding insulin (5.0 µg/ml), hydrocortisone (0.45 µg/ml), epidermal growth factor (EGF) (10.0 ng/ml), cholera toxin (10⁻⁹ M) and fetal bovine serum (10%), this is the same hereinafter) was added to dilute the cell solution, and the system was subjected to centrifugation (10°C or less, 1,500 rpm, 5 minutes). After the centrifugation, the supernatant was removed, 10 ml of FAD medium was added to the system, and pipetting was repeated until the cellular masses disappeared.

The number of the obtained cells was calculated by a cytometer, and the cell concentration was adjusted to 2.5 x 10⁵ cells/ml with FAD medium,

The cells were spread in a 75 cm³-flask coated with I-type collagen, and they were cultured overnight at 37°C and 5% CO₂.

After the culturing, the system was washed twice with 10 ml of PBS (-), 2 ml of PBS (-) containing 0.25% trypsin was added, and the mixture was incubated at 37°C and 5% CO₂ for 4 minutes. Then, 2 ml of fetal bovine serum (FBS) was added to the system, the mixture was once gently shaken, and the supernatant was removed to remove the fibroblasts contaminating the system.

Further, 15 ml of KGK medium [Keratinocyto growth medium: a medium obtained by adding bovine pituitary extract (BPE) (0.4 % by volume), insulin (0.5 µm/ml), hydrocortisone (0,5 µm/ml) and h-EGF (0.1 ng/ml) to KGM medium (modified MCDB153 medium (made by CLONE-TICS)), this is the same hereinafter] was added to the system, and the mixture was cultured at 37°C and 5% CO₂ for 3 days.

The fibroblasts contamination rate (FB contamination rate) of a culturing flask in which the hair follicular epitheliocytes obtained by the above step were spread was measured (3,000 magnifications, 5 visual fields), and the cells having a FB contamination rate of 3% or more were excluded from the subjects of the assay.

The system was washed twice with 10 ml of PBS (-), 2 ml of PBS (-) containing 0.25% trypsin was added, and incubation was carried out at 37°C for 3 minutes. Then, in order to remove fibroblasts from the system utilizing difference in reactivity with trypsin between epitheliocytes and fibroblasts, trypsin was removed. 2 ml of PBS (-) containing 0.25% trypsin was added again, and the mixture was shaken at 37°C and 20 rpm for 5 minutes.

Then, peeling of the cells was confirmed under a microscope, 10 ml of DMEM medium containing 10% FBS was added, pipetting was carried out in a 50-ml centrifugal tube, and the system was subjected to cetrifugation at 1.500 rpm for 5 minutes.

The supernatant was removed, 20 ml of KGM medium was added, and pipetting was carried out until the cellular masses disappeared.

The suspension was filtered by Cell Strainer (100 µm, made by NALGENE), and put in a 50-ml centrifugation tube. The number of live cells in the suspension was calculated by a cytometer, and KGM medium was added to the system to adjust the cell concentration in the system to 5.0 x 10⁴ cells/ml.

Then, the cell suspension was spread on a 96-well plate (plate coated with I-type collagen: made by FALCON) at a rate of 0.2 ml/well (1.0 x 10⁴ cells/well), and the system was left alone at room temperature for about 20 minutes until the cells sank on the bottom of the wells.

Thereafter, culturing was carried out at 37°C and 5% CO₂ for one day to give desired human cultured hair follicular epitheliocytes.

### 3. Preparation of test media

0.2% DMSO solution of the methanol extract (dried matter) of Croton birmanicus obtained in the above Preparation 1 was added to 1.000 volumes of modified MCDB153 medium (made by CLONETICS) [extract concentration: 2.0 x 10⁻⁴ % (DMSO 0.1%)].

These media to which the target substance was added were added to KBM medium containing 0.1% DMSO to dilute the concentration of the target substance so as to be 1.0 x 10⁻⁸ to 1.0 x 10⁻⁵ % (Samples 11 to 14). Likewise, a control sample not containing the medium to which the target substance was added was prepared.

### 4. Replacement with the media to which the target substance was added

In the above A and B, the KGM media in the 96-well plate used for preparation of human cultured hair follicular epitheliocytes and rat cultured hair follicular epitheliocytes were replaced with the media to which the target substance was added or the control medium (200 µl/well), prepared in the above 3, respectively, followed by culturing at 37°C and 5% CO₂ for 2 days.

The medium replacement was carried out by removing the KGM medium in each of the wells by an aspirator taking care that the cells adhering onto the bottom do not get hurt, and then immediately adding the medium to which the target substance was added or the like from both edges of the well.

### 5. Assay of cell proliferation

Alamar blue (made by ALAMAR BIOSCIENCE) was added in an amount of 1/10 based on the amount of the medium (volume), and the mixture was incubated at 37°C (5% CO₂) for 6 hours.

After the incubation, absorbance was measured at 570 nm and 595 nm using Micro plate reader (made by BIO RAD). According to the attached operation manual, the reduction rate of alamar blue was calculated based on the measurement results of absorbance. Since this reduction rate correlates with cell number, the cell proliferation rates in the control and test sample systems were compared.

### 6. Results

The above proliferation acceleration indexes in the assayed Croton birmanicus extracts (Samples 11 to 14) are shown in the following Table 5.

**Table 5**

| Sample | Proliferation acceleration rate (%) | Effect |
|---|---|---|
| Sample 11 (1.0 x 10⁻⁸ %) | 5.3±6.3* | effective |
| Sample 12 (1.0 x 10⁻⁷%) | 13.5±5.2** | effective |
| Sample 13 (1.0 x 10⁻⁶ %) | 15.5±3.1** | effective |
| Sample 14 (1.0 x 10⁻⁵ %) | 25.6±5.1** | effective |
| Control sample | 1.2±2.7 | noneffective |

| | | |
|---|---|---|
| *; significant difference p<0.05 | | |
| **; significant difference p<0.01 | | |

As apparent from Table 5, the Croton birmanicus extract of the invention showed a significant follicular epitheliocyte proliferation accelerating effect in the human cultured follicular epitheliocyte activation test.

### ⑤ Actual use test

The present invention sample example 15 Hair tonic

| | |
|---|---|
| (1) Croton birmanicus extract (Preparation 1) | 1.0 |
| (2) Propylene glycol | 5.0 |
| (3) Sodium hyaluronate | 0.01 |
| (4) 75% Ethanol | balance |

The effects of the hair tonic prepared in the above formulation in actual use on symptoms such as dandruff, hair generation and alopecia were examined. The hair tonic was administered to 10 males (age 25-55) showing symptoms such as dandruff, hair generation and alopecia, once or twice a day, 1 to 3 ml per each administration, over a period of 4 months, and it was tested for dandruff preventing effect, hair generation accelerating effect and alopecia preventing effect, according to the following criteria. The results are shown in Table 6.

### Evaluation criteria

(1) Dandruff preventing effect test
   Noneffective:
      No improvement was observed in spite of the treatment.
   Effective:
      Generation of dandruff was decreased.
   Remarkably effective:
      Generation of dandruff was stopped.
(2) Hair generating effect test
   Noneffective:
      No improvement was observed in spite of the treatment.
   Effective:
      Generation of hair was observed in 2/3 or more of the alopecia part.
   Remarkably effective:
      Hair comes out on the whole alopecia part.
(3) Alopecia effect test
   Noneffective:
      No improvement was observed in spite of the treatment.
   Effective:
      Progress of alopecia decreased.
   Remarkably effective:
      Alopecia was stopped.

**Table 6**

| Subject | Age | Dandruff | Hair generation | Alopecia |
|---|---|---|---|---|
| 1 | 43 | remarkably effective | effective | effective |
| 2 | 32 | remarkably effective | effective | effective |
| 3 | 45 | remarkably effective | effective | effective |
| 4 | 37 | effective | effective | noneffective |
| 5 | 41 | noneffective | effective | effective |
| 6 | 28 | effective | remarkably effective | remarkably effective |
| 7 | 25 | noneffective | effective | effective |
| 8 | 55 | effective | noneffective | effective |
| 9 | 33 | effective | effective | effective |
| 10 | 27 | noneffective | effective | remarkably effective |

As apparent from Table 6, this hair tonic exerts excellent effect of hair generation acceleration, dandruff prevention and alopecia prevention.

Formulation examples in various dosage forms of the composition for the head according to the invention are shown below as examples.

### Example 1 Shampoo

| | |
|---|---|
| Cocoylmethyltaurine sodium | 2.0 |
| Polyoxyethylene (8 mol) oleyl alcohol | 2.0 |
| Lauric acid diethanolamid | 4.0 |
| Ethylene glycol fatty acid ester | 1.0 |
| Glycerol | 0.2 |
| Menthol | 0.1 |
| Croton birmanicus extract (Preparation example 1) | 1.0 |
| Perfum | appropriate amount |
| Propylene glycol | 2.0 |
| Purified water | balance |

### Example 2 Hair treatment

| | |
|---|---|
| Liquid paraffin | 25.0 |
| Stearic acid | 5.0 |
| Cetanol | 5.0 |
| Sorbitan monooleat | 2.0 |
| Polyoxyethylene sorbitan monooleat | 3.0 |
| Croton birmanicus extract (Preparation example 1) | 0.5 |
| 1,3-butylene glycol | 5.0 |
| Antiseptic | appropriate amount |
| Purified water | balance |

### Example 3 Hair tonic

| | |
|---|---|
| Croton birmanicus extract (Preparation example 1) | 1.0 |
| Stearyldimethylamine oxid | 0.5 |
| Hardened castor oil ethylene oxide (40 mol) adduct | 1.0 |
| 95% Ethanol | 54.0 |
| Deionized water | balance |

### (Preparation process)

Deionized water was added to 95% ethanol, then hardened castor oil ethylene oxide (40 mol) adduct and stearyldimethylamine oxide were added thereto, the dried extract was added, and the mixture was stirred to give a solution.

### Example 4 Hair tonic

| | |
|---|---|
| Glycerol | 2.0 |
| L-menthol | 0.1 |
| Croton birmanicus extract (Preparation example 1) | 2.0 |
| 95% Ethanol | 54.0 |
| Perfum | 0.5 |
| Deionized water | balance |

### (Preparation process)

Glycerol, L-menthol, perfume and the dried extract were added to 95% ethanol, the mixture was stirred to give a solution, and deionized water was added.

### Example 5 Hair tonic

| | |
|---|---|
| Sodium N-cocolauryl-β-aminopropionat | 0.2 |
| Croton birmanicus extract (Preparation example 1) | 0.001 |
| Sodium dodecylbenzenesulfonat | 0.5 |
| Hardened castor oil ethylene oxide (40 mol) adduct | 1.0 |
| 95% Ethanol | 54.0 |
| Deionized water | balance |

### (Preparation process)

Deionized water was added to 95% ethanol, then hardened castor oil ethylene oxide (40 mol) adduct, stearyldimethylamine oxide and sodium N-coco palm lauryl-β-aminopropionate were added thereto, the dried extract was added, and the mixture was stirred to give a solution.

### Example 6 Hair tonic

| | |
|---|---|
| Glycerol | 2.0 |
| L-Menthol | 0.1 |
| Active fraction A obtained from the Croton birmanicus extract | 0.1 |
| 95% Ethanol | 54.0 |
| Perfum | 0.5 |
| Deionized water | balance |

### (Preparation process)

Glycerol, L-menthol, perfume and Active fraction A were added to 95% ethanol, the mixture was stirred to give a solution, and deionized water was added.

### Example 7 Hair tonic

| | |
|---|---|
| Hinokitiol | 0.1 |
| Swertia Herb extract | 1.0 |
| Vitamin B6 | 0.2 |
| Vitamin | 0.01 |
| Menthol | 0.2 |
| Salicylic acid | 0.1 |
| Active fraction A obtained from the Croton birmanicus extract | 0.1 |
| Surfactant | 0.1 |
| Propylene glycol | 2.0 |
| 70% Ethanol | balance |

### Example 8 Hair tonic

| | |
|---|---|
| Hinokitiol | 0.1 |
| Swertia Herb extract | 1.0 |
| Vitamin B6 | 0.2 |
| Vitamin | 0.01 |
| Menthol | 0.2 |
| Salicylic acid | 0.1 |
| Croton birmanicus extract (Preparation example 1) | 1.0 |
| Surfactant | 0.1 |
| Propylene glycol | 2.0 |
| 70% Ethanol | balance |

## Claims

1. A hair-revitalizing composition comprising an extract from a plant being classified as the genus Croton of the family Euphorbiaceae in an amount effective for revitalization of human hair, and cosmetically or dermatologically acceptable other additives.

2. The hair-revitalizing composition according to claim 1 wherein the plant being classified as the genus Croton is Croton birmanicus Muell. Arg.

3. The hair-revitalizing composition according to claim 1 wherein the extract is an extract obtained by carrying out the extraction using one or two or more selected from the group consisting of water-miscible organic solvents.

4. A composition for activating hair dermal papilla cells of human hair comprising an extract from a plant being classified as the genus Croton of the family Euphorbiaceae in an amount effective for activating the hair dermal papilla cells, and cosmetically or dermatologically acceptable other additives.

5. The composition according to claim 4 wherein the plant being classified as the genus Croton is Croton birmanicus Muell. Arg.

6. A composition for lengthen the anagen in the hair cycle of human hair comprising an extract from a plant being classified as the genus Croton of the family Euphorbiaceae in an amount effective for lengthen the anagen, and cosmetically or dermatologically acceptable other additives.

7. The composition according to claim 6 wherein the plant being classified as the genus Croton is Croton birmanicus Muell. Arg.

8. Use of an extract from a plant being classified as the genus Croton of the family Euphorbiaceae as an effective ingredient, for preparation of hair revitalizing compositions.

9. Use of an extract from a plant being classified as the genus Croton of the family Euphorbiaceae as an effective ingredient, for preparation of compositions activating hair dermal papilla cells of human hair, or lengthening the anagen in the hair cycle.

10. The use according to claim 8 or 9 wherein the plant being classified as the genus Croton is Croton birmanicus Muell. Arg.

11. An extract from a plant being classified as the genus Croton of the family Euphorbiaceae, for use as an effective ingredient of hair revitalizing compositions.
